# FASCICULE DE BREVET EUROPEEN

(11) **EP 2 037 975 B1**
(45) Date de publication et mention de la délivrance du brevet: **18.06.2014**
(21) Numéro de dépôt: 07729978.2
(22) Date de dépôt: 07.06.2007
(51) Int. Cl.: A61L 27/24, A61L 27/00

(54) **TUBES DE COLLAGENE**
KOLLAGENRÖHREN
COLLAGEN TUBES

(30) Priorité: 22.06.2006 FR 0605610; 22.03.2007 US 907142 P
(43) Date de publication de la demande: 25.03.2009
(73) Titulaire: Orthomed, 06640 Saint Jeannet (FR)
(72) Inventeur: GAGNIEU, Christian, F-69680 Chassieu (FR); GUYOT, Vincent, 69003 Lyon (FR)
(74) Mandataire: Hughes, Andrea Michelle
(86) Numéro de dépôt international: PCT/EP2007/055614
(87) Numéro de publication internationale: WO 2007/147739

(56) Documents cités:
- EP-A1- 0 943 345
- WO-A-82/03764
- US-A- 3 562 820
- US-A- 5 207 705

## Description

La présente invention concerne des tubes, faisceaux de tubes et associations de tubes et de faisceaux, composés de collagène, destinés à être utilisés en biologie et médecine comme support de croissance et/ou de différenciation de structures cellulaires, et en particulier en chirurgie pour favoriser la repousse de nerfs sectionnés.

L'invention concerne également un procédé de fabrication de films de collagène continus, cylindriques et coaxiaux, dont la succession constitue les parois desdits tubes.

Plusieurs techniques ont été développées pour réparer les lésions nerveuses et en particulier les nerfs sectionnés. Une technique courante consiste à poser chirurgicalement un tube reliant les deux extrémités du nerf sectionné, pour guider la repousse de la partie proximale du nerf vers la partie distale afin de permette leur jonction.

Pour créer un tube destiné à favoriser la repousse nerveuse, de nombreux polymères ont été testés comme par exemple les silicones, l'acide polyglycolique, ou les polymères d'acide lactique, mais aucun de ces composés ne répond aux exigences complexes de la repousse d'un nerf. En particulier il est préférable que le tube soit composé d'une matière résorbable.

Les praticiens ont défini des critères auxquels les tubes doivent répondre pour permettre une repousse optimale des nerfs sectionnés, en particulier:
- Les tubes doivent être constitués d'un matériel biocompatible et résorbable après un temps nécessaire à la mise en place et au développement des processus de croissance et de maturation des axones.
- Les tubes doivent présenter une résistance mécanique suffisante pour s'opposer aux pressions externes, mais être assez souples pour ne pas rentrer en conflit avec le nerf.
- L'extérieur et l'intérieur du tube doivent être lisses pour ne pas blesser les tissus environnants, ni les extrémités du nerf.
- La paroi du tube doit avoir une porosité faible, permettant uniquement la circulation des facteurs trophiques mais non celle des cellules.
- Les tubes doivent pouvoir être stérilisés

Le collagène est un matériel particulièrement bien adapté pour les implants chirurgicaux ; il présente des propriétés physico-chimiques et biologiques variées qui font de lui un matériau de premier choix pour la confection de tubes répondant aux caractéristiques listées ci-dessus. Le collagène est biocompatible et résorbable. Il est possible de moduler finement sa vitesse de résorption, ainsi que de lui associer des facteurs favorisant la repousse nerveuse. De plus, il présente une faible antigénicité, un rôle dans la croissance et la différenciation cellulaires, ainsi qu'un fort pouvoir hémostatique.

Les molécules de collagène sont des protéines animales situées dans la matrice extracellulaire qui possèdent dans leur structure un ou plusieurs domaines en triple hélice. La triple hélice est obtenue par association de trois chaînes alpha composées chacune de 1050 acides aminés. A l'extrémité des chaînes, des zones non hélicoïdales d'une quarantaine d'acides aminés, les télopeptides permettent l'association des molécules de collagène entre elles. Cet arrangement ordonné des macromolécules entre elles permet la formation de fibres.

Le collagène est extrait des tissus sources par des procédés bien connus de l'homme du métier. Il existe des collagènes de plusieurs types et de différents niveaux de structuration.
- Le collagène est dit natif lorsque l'ensemble de la structure qu'il adopte dans les tissus (triple hélice et télopeptides) est conservé à l'extraction.
- Le collagène peut être clivé de façon enzymatique ou chimique au niveau des télopeptides : le collagène est alors appelé atelocollagène.
- Lorsque les trois chaînes alpha de la triple hélice sont séparées par dénaturation (chauffage par exemple), le collagène est dit dénaturé.

Différents types de collagène ont été mis en évidence et certains ont été isolés et produits industriellement, essentiellement le type I, II, et le type IV.

Suivant le type de collagène utilisé, les techniques de préparation de la solution de collagène et de fabrication du tube, on obtiendra des produits présentant des caractéristiques physiques, physicochimiques et métaboliques différentes.

De nombreux documents décrivent des tubes de collagène destinés à favoriser et guider la repousse d'un nerf.

Le brevet EP 0 156 740 (US 4,814,120) décrit un procédé de fabrication de tubes de collagène pouvant être utilisés dans le domaine des prothèses vasculaires et des sutures nerveuses. Ces tubes sont constitués d'une seule couche de collagène.

Les brevets US 4,963,146 et US 5,026,381 décrivent un tube pour la régénération de nerfs sectionnés, constitué d'au moins deux couches de collagène I, présentant des perméabilités différentes, dont une couche extérieure poreuse.

Le brevet US 6,090,117 revendique un tube permettant de régénérer une portion de nerf manquante. La membrane de ce tube est recouverte de deux couches intérieure et extérieure, composées de gélatine ou de collagène. Ce tube favorise l'infiltration des capillaires sanguins. Le tube est rempli d'un « corps » de collagène, comprenant des cavités, elles-mêmes remplies de gel-matrice composé de collagène, laminine, proteoglycans et facteurs de croissance.

Le brevet US 6,716,225 (demande WO 2003/011149) décrit une matrice tubulaire composée de collagène. La matrice a un diamètre intérieur compris entre 0,1 et 10 mm. La paroi extérieure de cette matrice est caractérisée par sa rugosité.

Le brevet US 6,953,482 décrit un instrument utilisé pour favoriser la régénération nerveuse, composé d'un support en forme de tube, d'une matrice sous forme d'éponge remplissant le tube, et d'une structure tubulaire constituée de fibres de collagène, permettant de guider la repousse du nerf.

La demande de brevet US 2004/0170664 décrit un tube destiné à être utilisé en régénération neuronale, composé d'une paroi externe résorbable, lisse et non-poreuse. Cette paroi est composée d'un unique film de collagène replié sur lui-même, les deux extrémités étant soudées ou collées. Le tube peut être rempli d'une matrice de collagène.

Le document US 3.562.820 décrit une succession de couches de muqueuses collées les unes aux autres par une colle de collagène fibreux. Les couches sont un mélange composite non homogène.

L'invention décrite dans le document WO82/03764 est constituée d'une pluralité de couches contenant du collagène en association avec des cellules vivantes responsables de l'arrangement moléculaire au collagène.

Le document EP 0943345 décrit une succession de couches de collagène non tissé formées aléatoirement au moment de la congélation et de la déshydratation sous vide.

Le document US 5.207.705 décrit une succession de couches constituées d'un mélange de mousse de polyuréthane auquel on a ajouté du collagène au moment de la réticulation du polyuréthane.

Les tubes décrits dans les documents de l'état de la technique présentent divers inconvénients.

Si la porosité de la membrane externe du tube est trop grande, des cellules telles que les fibroblastes peuvent pénétrer à l'intérieur du tube, selon un phénomène « d'envahissement cicatriciel ». Cet envahissement est néfaste à la repousse du nerf.

Si les tubes présentent une paroi rugueuse, celle-ci peut blesser les tissus environnants. De même la présence de zones de soudure ou collage sur la membrane externe est un inconvénient car ces aspérités sont irritantes.

D'une manière générale, la présence de matrice dans les tubes ne permet pas de guider efficacement le nerf en repousse.

Enfin, du fait de leur mode de préparation, ces tubes sont dans la plupart des cas très onéreux.

La présente invention propose donc un nouveau type de tube de collagène. destiné à être utilisé pour la régénération d'un nerf, dans lequel est inséré un faisceau de tubules, soudés entre eux, permettant de guider individuellement la progression des fascicules d'axones, composants du nerf. Chaque tube et tubule est constitué d'une succession de films de collagène continus, cylindriques et coaxiaux, et de préférence présentant sensiblement la même porosité.

### DESCRIPTlON DE L'INVENTION

La présente invention concerne un tube de collagène pour la régénération d'un nerf caractérisé en ce qu'il comprend une paroi constituée par une succession de films de collagène continus. cylindriques circulaires et coaxiaux.

Par film continu on entend que le film est d'une seule pièce et ne contient pas de zone de soudure ou de collage. Ces films sont en forme de tube sensiblement cylindrique. Le terme « tube cylindrique » désigne tous cylindres issus d'une directrice, avantageusement d'une directrice circulaire. Le qualificatif coaxial indique que tous les films assemblés partagent un même axe, qui est l'axe du tube. Ces films sont avantageusement transparents, homogènes et ne contiennent peu ou pas d'agrégats. Ces films sont préférentiellement non poreux, c'est-à-dire qu'il n'y a pas de porosité supérieure à 1 µm. La diffusion de molécules de poids moléculaire d'environ 70 kDa à travers la paroi du tube est possible. Le temps moyen de résorption du tube est contrôlable et variable selon les traitements.

Avantageusement, chaque film de collagène constituant la paroi présente une épaisseur comprise entre 0,5 et 4µm.

L'invention se rapporte plus particulièrement à un tube de collagène, dont la paroi est constituée par une succession d'au moins 5 films de collagène continus, cylindriques et coaxiaux. De préférence la paroi est constituée par une succession de 5 à 30 films, et en particulier de 10 à 15 films.

L'homme du métier saura adapter le nombre de films constituant la paroi du tube en fonction des propriétés qu'il entend lui donner : résistance à la traction, souplesse, épaisseur, ces caractéristiques étant choisies selon l'utilisation envisagée.

Les films assemblés pour constituer la paroi d'un tube peuvent être tous de la même composition. Selon un autre aspect de l'invention, les films constituant une même paroi peuvent présenter des compositions différentes, c'est-à-dire être composés de différents types ou mélanges de collagènes. En particulier les films constituant les couches intérieures et extérieures peuvent être composés de différents mélanges de collagène.

Les films constituant les parois peuvent contenir du collagène I, du collagène III, ou un mélange des deux. Le mélange peut comprendre toutes les proportions relatives possibles de collagènes I et III. La composition préférée est un mélange comprenant 85 à 100% de collagène I et 0 à 15% de collagène III.

Selon un autre mode de réalisation de l'invention, les films peuvent comprendre du collagène IV, soit pur, soit associé à d'autres types de collagène, dans toutes les proportions relatives possibles. La composition préférée est un mélange comprenant de 1 à 100% en poids de collagène IV et de 0 à 99% en poids de collagène I, ou d'un mélange de collagène I + III dans les proportions décrites ci-dessus.

De manière avantageuse, les films constituant la partie la plus interne de la paroi comprennent du collagène IV, les autres films plus externes étant constitués de collagène I ou d'un mélange de collagènes I et III.

Les collagènes utilisés peuvent être issus de plusieurs espèces, en particulier provenir de l'espèce bovine ou porcine.

Selon un mode préféré de réalisation, le collagène utilisé est de l'atelocollagène, c'est-à-dire un collagène qui a subi un clivage au niveau des télopeptides (Rousseau & Gagnieu, Biomaterials, 2002).

Selon un autre aspect de l'invention, le collagène constituant les parois des tubes est réticulé. La réticulation se fait par les techniques classiques connues de l'homme du métier. Elle peut s'effectuer par exemple sous l'action de radiations. Préférentiellement la réaction de réticulation sera effectuée par trempage du tube de collagène dans une solution de formaldéhyde, à une concentration comprise entre 0,01 et 2%, pendant un temps compris entre 1 minute et 72 heures, à un pH compris entre 3 et 9,5, ces facteurs étant adaptés selon le taux de réticulation désiré.

Le tube de collagène selon l'invention peut être utilisé pour toutes sortes, d'application. Il est en particulier destiné à être utilisé dans la chirurgie neuronale, pour guider et protéger un nerf sectionné pendant sa repousse.

Pour cette application particulière, la section du tube de collagène est adaptée à la section du nerf qui doit être guidé dans sa repousse : idéalement le diamètre intérieur du tube doit être égal à la section du nerf à régénérer.

De façon générale, le diamètre intérieur du tube de collagène selon l'invention est compris entre 50 µm et 10 mm. Préférentiellement il est compris entre 1 et 7 mm.

L'invention concerne également des tubes de faible diamètre, ci-après nommés tubules, qui présentent un diamètre intérieur inférieur ou égal à 500 µm, et qui est préférentiellement compris entre 50 et 200 µm, et de préférence de 100 µm.

L'invention se rapporte également à un faisceau de tubes de collagène caractérisé en ce qu'il comprend au moins deux tubes selon l'invention, alignés en parallèle et soudés les uns aux autres.

De manière préférée, les tubes composant le faisceau sont tous de la même longueur et ont le même diamètre interne.

Selon un aspect préféré de l'invention, les tubes composant le faisceau présentent un diamètre intérieur inférieur à 500µm, et plus préférentiellement un diamètre intérieur compris entre 50 et 200 µm. Ils sont alors nommés « tubules ».

Selon un mode préféré de réalisation de l'invention, la soudure des tubes ou tubules est réalisée par fusion partielle des membranes externes desdits tubes. L'homme du métier saura déterminer les paramètres optimaux de la réaction de fusion pour obtenir une fusion partielle des membranes externes.

Le faisceau peut être composé d'un nombre de tubes ou tubules compris entre 2 et 60, et en particulier être composé de 2, 3, 4, 5, 10, 15, 20, 30, 40, 50 ou 60 tubules, en fonction du diamètre du faisceau que l'on désire obtenir.

Ce faisceau est également caractérisé en ce qu'il est enveloppé d'une gaine de collagène. Cette gaine a une épaisseur comprise entre 1 et 3 µm. Cet enrobage du faisceau permet de lui donner une surface lisse et homogène, ce qui facilite l'insertion du faisceau dans un tube, le cas échéant. Cette gaine est préférentiellement composée majoritairement de collagène de type I.

Le faisceau ainsi constitué est avantageusement soumis à une réaction de réticulation, après assemblage et soudure des tubules entre eux, puis gainage par un film de collagène. La réticulation peut être effectuée selon toutes les techniques classiques connues de l'homme du métier, et sera en particulier réalisée au moyen d'une solution de formaldéhyde tel que décrit précédemment.

La présente invention concerne également une association de tubes caractérisée en ce qu'elle comprend un tube selon l'invention, dans lequel est inséré longitudinalement au moins un faisceau de tubes selon l'invention.

Les diamètres respectifs du faisceau et du tube sont adaptés pour pouvoir être combinés. Le ou les faisceaux sont insérés dans le tube à l'état sec.

Les tubes et tubules selon l'invention peuvent présenter toutes les longueurs possibles ; en général on prépare un tube de longueur appropriée que l'on découpe ensuite à la longueur désirée, par des techniques classiques connues de l'homme du métier.

Dans le cas où l'association selon l'invention est destinée à être utilisée en régénération neuronale, la longueur du tube et du faisceau sera adaptée à la longueur de la section de nerf manquante.

En général les nerfs sectionnés présentent un manque de substance nerveuse de l'ordre de quelques centimètres, qui peut atteindre 15 cm. En ce qui concerne les nerfs collatéraux des doigts, la longueur de la substance manquante est de 2.5 à 3 cm. Pour les reconstructions de nerfs de plus gros diamètre, comme le nerf médian ou lors d'atteintes du plexus brachial, les pertes de substance peuvent être de 10 à 15 cm.

De manière préférée, la longueur du tube est comprise entre 5 mm et 10 cm.

Pour que le tube ou l'association de tubes selon l'invention protège efficacement la zone à régénérer ainsi que les deux extrémités du nerf à réparer, il est préférable que le tube soit plus long que la zone à régénérer. Idéalement la longueur du tube utilisé est supérieure à la longueur de la perte de substance nerveuse pour permettre, en particulier, la suture des terminaisons nerveuses sectionnées dans le tube. Cette longueur supplémentaire est avantageusement d'environ 6 mm, ce qui laisse 3 mm de chaque côté pour permettre la suture et assurer une bonne protection des extrémités.

Selon un aspect préféré de l'invention, l'association tube / faisceau selon l'invention est caractérisée en ce que la longueur du tube est supérieure à la longueur du faisceau; en particulier, le faisceau aura la longueur de la perte de substance nerveuse, et le tube aura une longueur supérieure pour assurer la protection de la suture et des extrémités, préférentiellement 6 mm de plus, soit 3 mm de chaque côté de la zone à régénérer.

Lors de la pose chirurgicale de l'association selon l'invention au niveau de la section manquante de nerf, le tube contenant le faisceau de tubules sera suturé aux extrémités du nerf par le praticien. Quel que soit le nerf à reconstruire, celui-ci est d'abord disséqué à l'oeil nu puis sous microscope, les extrémités sont recoupées jusqu'à retrouver une zone "saine" au niveau des deux moignons, puis le tuteur est mis en place à l'aide de plusieurs points de fil de suture microscopique. Les sutures proximales et distales peuvent entre "renforcées" à l'aide de colle biologique.

La présente invention concerne aussi un procédé de fabrication d'un tube de collagène tel que défini précédemment, dans lequel le collagène est solubilisé dans un solvant approprié, la solution obtenue est déposée sur un support de forme cylindrique puis séchée, ces deux étapes étant répétées pour obtenir une succession de films de collagène continus, concentriques et coaxiaux.

L'homme du métier choisira le solvant adéquat pour dissoudre le collagène, dans le but d'obtenir un film de collagène le plus homogène possible, en particulier ne comprenant peu ou pas d'agrégat. Pour éviter la formation d'agrégat, il est important que le séchage de la solution de collagène après coulage, c'est-à-dire l'évaporation du solvant, s'effectue le plus rapidement possible.

Les solvants aqueux traditionnellement utilisés pour dissoudre le collagène ont une évaporation assez lente, ce qui favorise la formation d'agrégat. Au contraire, lorsqu'on dissout le collagène dans un solvant organique approprié, celui-ci peut être évaporé à une vitesse suffisante pour éviter la formation d'agrégats.

Selon un mode préféré de mise en oeuvre du procédé de l'invention, le solvant est un solvant polaire. Avantageusement, le solvant contient du glycérol. La dissolution du collagène dans cette solution s'effectue sous agitation pendant quelques heures.

De manière avantageuse, le solvant contient du méthanol, et est préférentiellement un mélange de méthanol et d'eau (méthanol 30-100%, eau 0-70% en volume) ou encore du méthanol pur à 98-100%. Toutes les formes de collagène peuvent être utilisées : collagène acido-soluble, collagène fibreux, atélocollagène ou collagène dénaturé. La forme préférée est l'atélocollagène à un taux de pureté élevé (environ 99%).

Ce procédé présente l'avantage que le temps de séchage est de 5 minutes pour un tube de diamètre intérieur de 1,5 mm ; cette valeur peut augmenter en fonction du diamètre du tube, mais l'incrémentation du temps de séchage sera de l'ordre de la minute. Pour un procédé équivalent utilisant une solution aqueuse, le temps de séchage serait supérieur à une heure.

Pour la fabrication de tubules, la solution de collagène dilué contient avantageusement un composé tensioactif, qui sera éliminé par le lavage ultérieur du tubule. Préférentiellement, on s'assure que la solution de collagène dissous ne présente pas d'agrégats de taille supérieure à 50 µm, par extrusion à travers un filtre.

Selon un mode préféré de mise en oeuvre du procédé, le support de forme cylindrique est un tube en polymère synthétique à surface lisse présentant un diamètre égal au diamètre intérieur souhaité pour le tube. Préférentiellement le support est en PTFE.

Le dépôt de la solution de collagène dissous s'effectue par immersion du support dans la solution. Entre chaque trempage, le tube est séché sous flux d'air dépoussiéré. Les étapes d'immersion du support et de séchage seront répétées autant de fois que l'on désire assembler de films ; en particulier les étapes seront répétées entre 5 et 30 fois pour obtenir un tube dont la paroi est constituée d'une succession de 5 à 30 films.

Le collagène des tubes sera ensuite préférentiellement soumis à une étape de réticulation. Le support cylindrique ne sera retiré qu'après cette étape de réticulation.

De manière préférée, le tube de collagène formé par la succession d'étapes trempage / séchage est ensuite immergé dans une solution de formol à pH contrôlé, puis dans de l'eau, puis dans une solution de glycine, puis dans l'eau, et enfin dans l'acétone, avant le démoulage des tubes.

L'invention se rapporte également à tout film de collagène susceptible d'être obtenu selon le procédé tel que précédemment décrit. Avantageusement, ce film présente les caractéristiques d'être transparent, parfaitement homogène et ne contenant pas ou peu d'agrégats.

Pour préparer un faisceau de tubules, ces derniers contenant toujours le support sont assemblés longitudinalement. Le faisceau de tubules ainsi obtenu est plongé dans un solvant adéquat permettant le gonflement et la dissolution partielle des couches les plus extérieures de chaque tubule. Le faisceau ainsi traité est soumis à une faible force longitudinale permettant un rapprochement des tubules et une fusion des couches en cours de solubilisation. Le séchage rapide sous flux d'air du faisceau assure la solidification des zones fusionnées et donc la soudure des tubules entre eux.

Après séchage, le faisceau est entouré d'un film de collagène, puis soumis à une étape de réticulation du collagène. Les moules sont retirés après la réticulation.

De façon avantageuse, la réticulation du collagène est effectuée de la manière suivante : immersion du faisceau dans une solution de formol à pH contrôlé comme préalablement décrit, puis immersion successive dans eau, glycine 0,1M, eau et acétone.

La présente invention concerne un tube de collagène ou un faisceau de tubes ou une association de tubes de collagène selon l'invention, pour son utilisation en chirurgie.

L'invention se rapporte également à l'utilisation d'un tube ou d'un faisceau de tubes ou d'une association de tubes, pour la préparation d'un dispositif chirurgical destiné à favoriser et guider la repousse d'un nerf sectionné.

Les tubes selon l'invention sont particulièrement adaptés à la neurochirurgie, car ils peuvent être utilisés pour la préparation d'un dispositif chirurgical permettant de favoriser et guider la repousse d'un nerf qui a été sectionné.

Le tube de collagène ou l'association de tubes selon l'invention pourront servir de « tuteurs » permettant à deux extrémités de nerf sectionné ou endommagé d'une quelconque façon de se rejoindre et se reconnecter. Cette technique peut être appliquée aussi bien à des nerfs moteurs qu'à des fibres sensitives.

### EXEMPLES

### Fabrication du tube entourant les faisceaux de tubules:

Une solution de collagène est préparée par dissolution sous agitation de 0,25g de collagène dans 50 ml de méthanol contenant du glycérol à une concentration correspondant à 50-100% de la concentration finale en collagène. La solution obtenue est homogénéisée par extrusions successives à travers des tamis de mailles 150 et 50µm.

Un moule cylindrique et rectiligne en PTFE de diamètre compris entre 500µm et 10 mm est plongé dans la solution de collagène et retiré après 2-15 secondes d'attente à une vitesse comprise entre 0,5 et 2 cm par seconde. Le moule enduit de solution de collagène est alors soumis à un mouvement de rotation dans un flux d'air filtré pendant 2 à 15 mn pour obtenir une évaporation homogène du solvant. Les opérations de trempage et séchage sont réalisées de 2 à 30 fois en fonction du nombre de couches de collagène désirées. Après le dernier trempage, le séchage final est effectué pendant 15 mn.

La réticulation du collagène est obtenue par immersion du moule supportant les couches de collagène dans un bain de formaldéhyde à 0.01-0.5% pendant un temps variant entre 5 minutes et 24h. Il est ensuite immergé successivement 5 minutes dans un bain d'eau, 5-60 minutes dans un bain de glycine 0.1M, 30 minutes dans un bain d'eau puis 5-15 minutes dans de l'acétone. Après évaporation de l'acétone 5-15 minutes dans un flux d'air filtré, le moule en PTFE est retiré pour donner un tube en collagène multicouches réticulé qui est placé au moins 16h dans une enceinte à dessiccation pour le séchage final jusqu'à un taux d'humidité inférieur ou égal à 15%. Selon ce procédé, des tubes de 3 à 25cm sont obtenus.

### Fabrication des tubules :

La solution de collagène est préparée comme précédemment mais contient en plus 0.5% (v/v) d'un produit tensioactif tel que le Tween 20. Cette solution est filtrée 3 fois sur tamis 50µm. les moules en PTFE de diamètre 50-200µm sont immergés en 1-5 secondes dans cette solution et le retrait est effectué à une vitesse comprise entre 3 et 10 centimètres par seconde. L'évaporation du solvant est réalisée par exposition du moule enduit de collagène 5 minutes sous flux d'air. L'opération peut est réalisée de 2 à 30 fois.

### Fabrication des faisceaux de tubules:

Les tubules supportés par leurs moules en PTFE sont réunis en faisceaux de 3 à 60 unités parallèles et les faisceaux sont ligaturés aux extrémités. Ces faisceaux sont alors immergés sans traction pendant 2 à 20 secondes dans un bain de méthanol, puis ils sont soumis à une faible traction longitudinale pour amener les tubules en contact étroit les uns avec les autres, cette opération étant suivie d'un séchage de 5 à 15 minutes sous flux d'air. Le faisceau obtenu est enduit de collagène par des cycles d'immersion/séchage (1 à 5) dans une solution méthanolique de collagène à la concentration de 0,1-0,5% contenant 0,25% de glycérol.

Les faisceaux obtenus sont réticulés, démoulés et séchés selon le procédé décrit dans la fabrication des tubes / enveloppes extérieures. Selon ce procédé, des faisceaux de 3 à 60 tubules sont obtenus.

### Association des tubes et faisceaux de tubules:

L'assemblage des deux éléments est réalisé à l'état sec. Le diamètre du tube/ enveloppe est supérieur au diamètre du faisceau qui est lié au nombre et au diamètre des tubules qu'il contient. Le faisceau est inséré longitudinalement dans l'enveloppe. Sa longueur peut être égale ou inférieure à celle de l'enveloppe.

### DESCRIPTION DES FIGURES

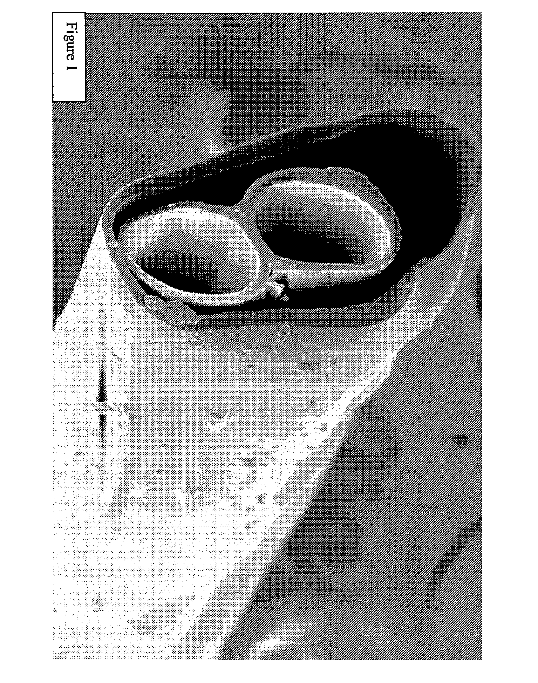

Figure 1 : Combinaison d'un tube de 500µm de diamètre et d'un faisceau de 2 tubes de 200µm de diamètre. Observation au microscope électronique à balayage.

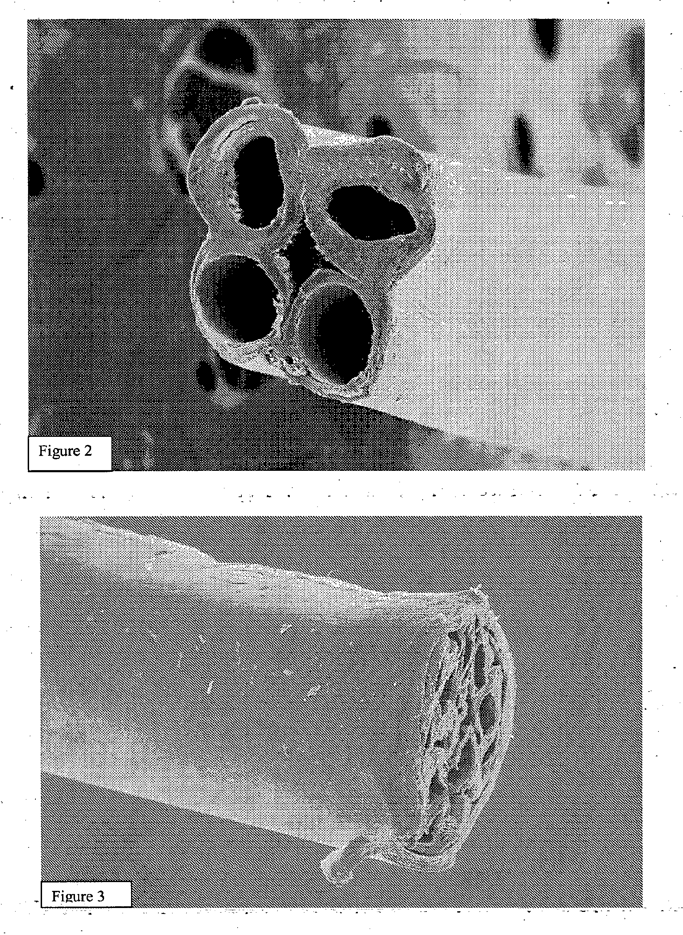

Figure 2 : Faisceau de quatre tubes de 200µm de diamètre. Observation au microscope électronique à balayage.
Figure 3 : Combinaison d'un tube de 500µm de diamètre et d'un faisceau de 10 tubes de 100µm de diamètre. Observation au microscope électronique à balayage.

## Revendications

1. Tube de collagène destiné à être utilisé pour la régénération d'un nerf **caractérisé en ce qu'**il comprend une paroi constituée par une succession de films de collagène continus, cylindriques essentiellement circulaires et coaxiaux.

2. Tube selon la revendication 1, **caractérisé en ce que** chaque film constituant la paroi a une épaisseur comprise entre 0,5 et 4 µm.

3. Tube selon l'une des revendications 1 ou 2, **caractérisé en ce que** la paroi est constituée par un assemblage d'au moins 5 films.

4. Tube selon l'une des revendications 1 à 3, **caractérisé en ce que** la paroi est constituée par un assemblage comprenant entre 5 et 30 films, de préférence de 10 à 15 films.

5. Tube selon l'une des revendications 1 à 4, **caractérisé en ce que** les films constituant la paroi comprennent du collagène I, du collagène III ou un mélange des deux.

6. Tube selon l'une des revendications 1 à 5, **caractérisé en ce que** les films constituant la couche intérieure de la paroi comprennent du collagène IV.

7. Tube selon l'une des revendications 1 à 6, **caractérisé en ce que** le collagène est de l'atelocollagène.

8. Tube selon l'une des revendications 1 à 7, **caractérisé en ce que** le collagène est réticulé.

9. Tube selon l'une des revendications 1 à 8, **caractérisé en ce que** le diamètre intérieur du tube est compris entre 50 µm et 10 mm

10. Faisceau de tubes de collagène **caractérisé en ce qu'**il comprend au moins deux tubes selon l'une des revendications 1 à 9, alignés en parallèle et soudés les uns aux autres.

11. Faisceau selon la revendication 10, **caractérisé en ce que** les tubes sont des tubules dont le diamètre intérieur est compris entre 50 et 200 µm.

12. Faisceau selon l'une des revendications 10 ou 11, **caractérisé en ce que** la soudure des tubes est réalisée par fusion partielle des membranes externes desdits tubes.

13. Faisceau selon l'une des revendications 10 à 12, **caractérisé en ce qu'**il est enveloppé d'une gaine composée de collagène.

14. Association de tubes de collagène **caractérisée en ce qu'**elle comprend un tube selon l'une des revendications 1 à 9, dans lequel est inséré longitudinalement au moins un faisceau de tubes selon l'une des revendications 10 à 13.

15. Association selon la revendication 14 **caractérisée en ce que** la longueur du tube est comprise entre 5 mm et 10 cm.

16. Association selon la revendication 15, **caractérisée en ce que** la longueur du tube est supérieure à la longueur du faisceau de tubes.

17. Procédé de fabrication d'un tube de collagène selon l'une des revendications 1 à 9, **caractérisé en ce que** le collagène est solubilisé dans un solvant approprié, la solution obtenue est déposée sur un support de forme cylindrique puis séchée sous flux d'air, ces deux étapes étant répétées pour obtenir une succession de films de collagène.

18. Procédé selon la revendication 17, **caractérisé en ce que** le solvant contient du méthanol.

19. Procédé selon la revendication 17, **caractérisé en ce que** le solvant est un mélange de méthanol et d'eau.

20. Procédé selon l'une des revendications 17 à 19, **caractérisé en ce qu'**il comprend une étape de réticulation du collagène avant de retirer le support.

21. Tube selon l'une des revendications 1 à 9, ou faisceau de tubes selon l'une des revendications 10 à 13, ou association de tubes selon l'une des revendications 14 à 16, pour leur utilisation en chirurgie.

22. Utilisation d'un tube selon l'une des revendications 1 à 9, ou d'un faisceau de tubes selon l'une des revendications 10 à 13, ou d'une association de tubes selon l'une des revendications 14 à 16, pour la préparation d'un dispositif chirurgical pour favoriser et guider la repousse d'un nerf sectionné.

## Patentansprüche

1. Kollagenrohr für das Wiederherstellen von einem Nerv, **dadurch gekennzeichnet, dass** es eine Wand aufweist, die aus einer Reihe zylinderförmiger, im Wesentlichen kreisförmiger und koaxialer, kontinuierlicher Kollagenfolien gebildet ist.

2. Kollagenrohr nach Anspruch 1, **dadurch gekennzeichnet, dass** jeder Film, der die Wand bildet, eine Dicke zwischen 0,5 und 4 µm aufweist.

3. Kollagenrohr nach einem der Ansprüche 1 oder 2, **dadurch gekennzeichnet, dass** die Wand durch eine Anordnung von wenigstens 5 Schichten gebildet ist.

4. Kollagenrohr nach einem der Ansprüche 1 bis 3, **dadurch gekennzeichnet, dass** die Wand durch eine Anordnung gebildet ist, die zwischen 5 und 30 Schichten, vorzugsweise zwischen 10 und 15 Schichten, aufweist.

5. Kollagenrohr nach einem der Ansprüche 1 bis 4, **dadurch gekennzeichnet, dass** die Filme, die die Wand bilden, Kollagen I, Kollagen III oder einer Mischung hiervon enthalten.

6. Kollagenrohr nach einem der Ansprüche 1 bis 5, **dadurch gekennzeichnet, dass** die Filme, die die innere Schicht der Wand bilden, Kollagen IV enthalten.

7. Kollagenrohr nach einem der Ansprüche 1 bis 6, **dadurch gekennzeichnet, dass** das Kollagen Atelokollagen ist.

8. Kollagenrohr nach einem der Ansprüche 1 bis 7, **dadurch gekennzeichnet, dass** das Kollagen vernetzt ist.

9. Kollagenrohr nach einem der Ansprüche 1 bis 8, **dadurch gekennzeichnet, dass** der Rohrinnendurchmesser 50 µm bis 10 mm beträgt.

10. Rohrbündel aus Kollagen, **dadurch gekennzeichnet, dass** es wenigstens 2 Kollagenrohre nach einem der Ansprüche 1 bis 9 aufweist, die parallel ausgerichtet und miteinander verschweißt sind.

11. Bündel nach Anspruch 10, **dadurch gekennzeichnet, dass** die Rohre Rohre mit einem Innendurchmesser von 50µm bis 200 µm sind.

12. Bündel nach einem der Ansprüche 10 oder 11, **dadurch gekennzeichnet, dass** das Verschweißen der Rohre durch ein teilweises Aufschmelzen der äußeren Membranen der Rohre bewirkt ist.

13. Bündel nach einem der Ansprüche 10 bis 12, **dadurch gekennzeichnet, dass** es von einer Hülle das Kollagen umhüllt ist.

14. Kombination von Kollagenrohren, **dadurch gekennzeichnet, dass** sie ein Rohr nach einem der Ansprüche 1 bis 9 aufweist, wobei mindestens ein Rohrbündel in Längsrichtung in eines nach einem der Ansprüche 10 bis 13 eingesetzt ist.

15. Kombination nach Anspruch 14, **dadurch gekennzeichnet, dass** die Länge des Rohres von 5 mm bis 10 cm beträgt.

16. Kombination nach Anspruch 15, **dadurch gekennzeichnet**, der die Rohrlänge größer als die Länge des Rohrbündels ist.

17. Verfahren zur Herstellung eines Rohrs das Kollagen nach einem der Ansprüche 1 bis 9, **dadurch gekennzeichnet, dass** das Kollagen in einem geeigneten Lösungsmittel gelöst, die erhaltene Lösung auf einen Träger mit zylindrischer Form aufgebracht und dann in einem Luftstrom getrocknet wird, wobei diese beiden Schritte wiederholt werden, um eine Reihe von Kollagenfolien zu erhalten.

18. Verfahren nach Anspruch 17, **dadurch gekennzeichnet, dass** das Lösungsmittel Methanol enthält.

19. Vorfahren nach Anspruch 17, **dadurch gekennzeichnet, dass** das Lösungsmittel ein Gemisch aus Methanol und Wasser ist.

20. Verfahren nach einem der Ansprüche 17 bis 19, **dadurch gekennzeichnet, dass** das Verfahren einen Schritt Vernetzen des Kollagens vor dem Entfernen des Trägers aufweist.

21. Rohr nach einem der Ansprüche 1 bus 9, oder ein Rohrbündel nach einem der Ansprüche 10 bis 13, oder eine Kombination mit einem Rohr nach einem der Ansprüche 14 bis 16 zur Verwendung in der Chirurgie.

22. Verwendung eines Rohrs nach einem der Ansprüche 1 bis 9, oder eines Rohrbündels nach einem der Ansprüche 10 bis 13, oder einer Kombination eines Rohres nach einem der Ansprüche 14 bus 16, zur Herstellung einer chirurgischen Vorrichtung zum Unterstützen und Führen des Wiederherstellens eines abgetrennten Nervs.

## Claims

1. Collagen tube to be used for the regrowth of a nerve **characterized in that** it comprises a wall formed by a series of cylindrical, mainly circular and coaxial, continuous collagen films.

2. Tube according to claim 1, **characterized in that** each film constituting the wall has a thickness between 0.5 and 4 µm.

3. Tube according to one of claims 1 or 2, **characterized in that** the wall is constituted by an assembly of at least 5 films.

4. Tube according to one of claims 1 to 3, **characterized in that** the wall is constituted by an assembly comprising between 5 and 30 films, preferably from 10 to 15 films.

5. Tube according to one of claims 1 to 4, **characterized in that** the films constituting the wall include collagen I, collagen III or a mixture thereof.

6. Tube according to one of claims 1 to 5, **characterized in that** the films constituting the inner layer of the wall comprise collagen IV.

7. Tube according to one of claims 1 to 6, **characterized in that** the collagen is atelocollagen.

8. Tube according to one of claims 1 to 7, **characterized in that** the collagen is crosslinked.

9. Tube according to one of claims 1 to 8, **characterized in that** the tube inside diameter is between 50 µm and 10 mm.

10. Tube bundle of collagen **characterized in that** it comprises at least two tubes according to one of claims 1 to 9, aligned in parallel and welded to each other.

11. Bundle according to claim 10, **characterized in that** the tubes are tubules whose inside diameter is between 50 and 200 µm.

12. Bundle according to one of claims 10 or 11, **characterized in that** the welding of the tubes is carried out by partial fusion of the outer membranes of said tubes.

13. Bundle according to one of claims 10 to 12, **characterized in that** it is enveloped by a sheath comprised of collagen.

14. Combination of collagen tubes **characterized in that** it comprises a tube according to one of claims 1 to 9, wherein at least one tube bundle is inserted longitudinally according to one of Claims 10 to 13.

15. Combination according to claim 14 **characterized in that** the length of the tube is between 5 mm and 10 cm.

16. Combination according to claim 15, **characterized in that** the tube length is greater than the length of the tube bundle.

17. Method for manufacturing a tube of collagen according to one of claims 1 to 9, **characterized in that** the collagen is solubilized in a suitable solvent, the solution obtained is deposited on a support of cylindrical shape and then dried in a stream of air, these two steps being repeated to obtain a series of collagen films.

18. Method according to claim 17, **characterized in that** the solvent contains methanol.

19. Method according to claim 17, **characterized in that** the solvent is a mixture of methanol and water.

20. Method according to one of claims 17 to 19, **characterized in that** it comprises a step of crosslinking the collagen before removing the support.

21. Tube according to one of claims 1 to 9, or bundle of tubes according to one of Claims 10 to 13, or combination of tubes according to one of claims 14 to 16, for use in surgery.

22. Use of a tube according to one of claims 1 to 9, or a bundle of tubes according to one of Claims 10 to 13, or a combination of tubes according to one of claims 14 to 16 for the preparation of a surgical device to assist and guide the regrowth of a severed nerve.
